Europäisches Patentamt

(19) European Patent Office

Office européen des brevets

(11) Publication number: **0 106 649**
**B1**

(12) EUROPEAN PATENT SPECIFICATION

(45) Date of publication of patent specification: **12.08.87**   (51) Int. Cl.⁴: **A 61 K 31/557**

(21) Application number: **83306130.2**

(22) Date of filing: **10.10.83**

(54) Prostaglandins and their use.

(30) Priority: **12.10.82 US 433587**

(43) Date of publication of application:
**25.04.84 Bulletin 84/17**

(45) Publication of the grant of the patent:
**12.08.87 Bulletin 87/33**

(84) Designated Contracting States:
**BE CH DE FR GB IT LI NL SE**

(56) References cited:
**US-A-3 927 213**
**US-A-3 954 833**
**US-A-4 081 553**

**PROSTAGLANDINS, supplement to vol. 21,
1981 M.J. RUWART et al.: "Protective effects of
16, 16-dimethyl PGE2 on the liver and kidney",
pages 97-102**

The file contains technical information
submitted after the application was filed and
not included in this specification

(73) Proprietor: **THE UPJOHN COMPANY**
**301 Henrietta Street**
**Kalamazoo, Michigan 49001 (US)**

(72) Inventor: **Robert, Andre**
**c/o The Upjohn Company 301 Henrietta Street**
**Kalamazoo Michigan 49001 (US)**
Inventor: **Elliott, George Algimon**
**c/o The Upjohn Company 301 Henrietta Street**
**Kalamazoo Michigan 49001 (US)**

(74) Representative: **Perry, Robert Edward et al**
**GILL JENNINGS & EVERY 53-64 Chancery Lane**
**London WC2A 1HN (GB)**

## Description

The present invention relates to the use of a renal cytoprotective prostaglandin for the manufacture of a medicament for preventing, in a mammal, renal papillary necrosis (RPN) induced by the administration of a non-steroidal anti-inflammatory compound (NOSAC).

RPN is a condition of the kidneys. Originally, RPN was thought to be a form of fulminating pyelonephritis; see Morales *et al*, Arch. Surg. 103:420 (1971). However, it has also been found to be associated with the administration of various analgesic compounds; see Spuhler *et al*, Z. Klin. Med. 151:1—50 (1953). RPN is characterised by morphological changes and renal failure, as a consequence of damage to the renal papillae. RPN has been associated with phenylbutazone (see Morales, *supra*); phenacetin (see Burry *et al*, Med. J. Aust., 1:31—36 (1974)); and aspirin, amidopyridine and indomethacin (see Arnold *et al*, Pathology 6:303—313 (1974)). RPN is thus a serious problem associated with the administration of a NOSAC.

The prostaglandins are derivatives of prostanoic acid. Known prostaglandins include 16,16-dimethyl-$PGE_2$ (see US—A—3903131) and 16,16-dimethyl-$PGF_{2\alpha}$ (see US—A—3954833).

Pharmacological agents which prevent necrotic changes in cells are known as cytoprotective agents. Numerous gastro-intestinal cytoprotective effects of the prostaglandins are known; see, for example, US—A—4081553, US—A—4083998 and US—A—4907603.

$PGE_2$ has been shown to protect against renal failure caused by glycerol in saline-loaded rats; see Papanicolaou *et al*, Clin. Sci. Mol. Med. 49:507 (1975). Ruwart *et al*, Prostaglandins 21 (Supplement):97—102 (1981), disclose that 16,16-dimethyl-$PGE_2$ protects the kidney from effects caused by administering carbon tetrachloride and α-naphthylisothiocyanate. US—A—3917828 discloses that certain prostaglandins are useful for the prevention of gastro-intestinal side-effects caused by NOSAC administration.

According to the present invention, a renal protective prostaglandin is used for the manufacture of a medicament for the prevention of RPN induced by NOSAC administration to a mammal. The term "prevention" means partial to total avoidance of kidney damage due to RPN.

For the purposes of this invention, those compounds which are useful as renal cytoprotective prostaglandins are those prostaglandins or prostaglandin analogues which are at least 1% as potent as 16,16-dimethyl-$PGE_2$ in effecting a reduction in renal papillary necrosis induced by mefenamic acid in the standard laboratory test set forth, below, in the Example.

16,16-Dimethyl-$PGE_2$ and 16,16-dimethyl-$PGF_{2\alpha}$ are preferred prostaglandins for use according to this invention. Other prostaglandins which are also useful for this purpose include those prostaglandins that exert a cytoprotective effect on the gastro-intestinal tract, as described, for example, in US—A—3917828, US—A—4081553 and US—A—4097603.

An important aspect of the utility of this invention is the determination of subjects who are particularly susceptible to RPN. Such subjects include those mammals, especially humans, who are receiving high doses of a particular NOSAC which is known to be particularly potent in causing RPN and who are not receiving other cytoprotective prostaglandin therapy.

Well-known side-effects of NOSAC administration are the developments of gastric lesions and gastro-intestinal distress. These gastro-intestinal side-effects from NOSAC administration stem from the fact that NOSAC are prostaglandin synthetase inhibitors. Among the NOSAC which are also prostaglandin synthetase inhibitors are indomethacin, aspirin, phenylbutazone, mefenamic acid, flufenamic acid, naproxen, 2-phenoxyphenylpropionic acid, (+)-3-chloro-4-cyclohexyl-α-methylphenylacetic acid and ibuprofen.

However, the gastro-intestinal side-effects of NOSAC administration are not experienced by approximately half of the subjects who receive such treatment. Thus, for example, many patients who are receiving large doses of NOSAC for anti-arthritic purposes do not experience any gastro-intestinal distress. Many of these patients may, however, be at risk for RPN. Such patients are prime subjects for the purposes of the present invention.

The current state of medical knowledge does not permit the precise prediction of persons particularly susceptible to RPN. However, all persons receiving NOSAC therapy are at risk for RPN, especially those who have a history of kidney disease or kidney injury or those receiving a NOSAC which is particularly potent in causing RPN. Experimentally, such NOSAC include fenoprofen, ibuprofen, flurbiprofen, indomethacin, mefenamic acid, naproxen and phenylbutazone. However, a prostaglandin is best used, according to the invention, prophylactically by administration to all persons who are at risk.

The present medicament can be applied for the treatment of various mammalian species. Humans are the most preferred subjects. With respect to non-humans, the present invention is particularly suitable for the treatment of domesticated animals such as cattle, horses, dogs, cats and swine.

Any convenient route of administration is employed. Oral administration is, for example, the preferred route for use in humans although parenteral (e.g. intravenous, intraperitoneal or intramuscular) administration may also be employed. US—A—3903297, columns 6—16, gives some appropriate and well-known means of administering the prostaglandins discussed herein.

The dosage regimen for the renal cytoprotective prostaglandin used in accordance with this invention will depend on a variety of factors, including the type, age, weight, sex and medical condition of the mammal, the dosage regimen of the NOSAC, the kind of NOSAC administered, and the renal cytoprotective

2

prostaglandin to be administered. It is within the skill of the attending physician or veterinarian to determine the class of subjects who are at risk from RPN, and to prescribe an effective preventive amount of the renal cytoprotective prostaglandin. In doing that, the physician or veterinarian would, by one procedure, start at a relatively low dose of the renal cytoprotective prostaglandin, e.g. 0.25 mg/kg/day to 0.1 µg/kg/day, and observe the response of the human or animal patient for a few days. The dose of the renal cytoprotective prostaglandin would then be adjusted downward or upward until the maximum effective dose is found. For example, the maximum needed dose is usually between 25 mg/kg/day and 15 µg/kg/day, although it may be necessary to exceed this dose occasionally, when the renal side-effects of NOSAC therapy are especially severe. Once the minimum effective dose of the particular renal cytoprotective prostaglandin is determined for a particular subject, it is advantageous to provide the subject with the dosage schedule which will provide a substantially uniform level of renal cytoprotective prostaglandin to the kidney.

The present invention is illustrated by the following Example.

Example

Single oral doses of mefenamic acid were given to rats over a dose range previously shown to produce RPN. Two prostaglandins, 16,16-dimethyl-$PGE_2$ and 16,16-dimethyl-$PGF_{2\alpha}$, were given orally by stomach tube twice daily for 4 days, beginning one hour before mefenamic acid administration. Doses of 800 and 1250 mg/kg of mefenamic acid, 0.2 mg/kg of 16,16-dimethyl-$PGE_2$ and 10 mg/kg of 16,16-dimethyl-$PGF_{2\alpha}$ were used.

Single oral doses of 800 mg/kg of mefenamic acid produced RPN in all rats. The incidence was reduced to 40% by 16,16-dimethyl-$PGE_2$ ($P<0.01$) and to 56% by 16,16-dimethyl-$PGF_{2\alpha}$ ($P<0.05$). At a dose of 1250 mg/kg of mefenamic acid, 45% of the animals died. Mortality was reduced to 18% in the groups given this dose of mefenamic acid plus either of the prostaglandins. The incidence of RPN was reduced from 91% (1259 mg/kg of mefenamic acid alone) to 73% (NS) and 55% (NS) when given mefenamic acid plus either 16,16-dimethyl-$PGE_2$ or 16,16-dimethyl-$PGF_{2\alpha}$, respectively. The intestinal lesions characteristic of NOSAC treatment (jejuno-ileal ulcers) produced by both dose levels of mefenamic acid were almost completely prevented by the two prostaglandins.

The results are summarised in the following Table. As can be seen, both prostaglandins significantly decreased the incidence of RPN induced by mefenamic acid.

**0 106 649**

TABLE 1
Treatment regimens and results

| Treatment | All animals RPN incidence | Survivors only | |
|---|---|---|---|
| | | RPN incidence | % RPN |
| **Regimen 1** (Preliminary study) | | | |
| Untreated | 0/3 | 0/3 | 0 |
| 1600 mg/kg MA | 10/10 | 4/4 | 100 |
| 1600 mg/kg MA+0.1 mg/kg $PGE_2$ | 5/6 | 2/3 | 67 |
| 1600 mg/kg MA+5 mg/kg $PGF_{2\alpha}$ | 5/6 | 5/5 | 100 |
| 2500 mg/kg MA | 9/10 | 0 | |
| 2500 mg/kg MA+0.1 mg/kg $PGE_2$ | 3/6 | 0 | |
| 2500 mg/kg MA+5 mg/kg $PGF_{2\alpha}$ | 6/6 | 0 | |
| **Regimen 2** | | | |
| Vehicle #122+5% ethanol | 0/11 | 0/11 | 0 |
| 800 mg/kg MA+5% ethanol | 11/11 | 10/10 | 100 |
| 800 mg/kg MA+$PGE_2$ (0.2 mg/kg) | 4/11 | 4/10 | 40 |
| 800 mg/kg MA+$PGF_{2\alpha}$ (10 mg/kg) | 6/11 | 6/11 | 55 |
| 1250 mg/kg MA+5% ethanol | 10/11 | 6/6 | 100 |
| 1250 mg/kg MA+$PGE_2$ (0.2 mg/kg) | 8/11 | 7/9 | 78 |
| 1250 mg/kg MA+$PGF_{2\alpha}$ (10 mg/kg) | 6/11 | 5/9 | 56 |
| Vehicle #122+$PGE_2$ (0.2 mg/kg) | 0/11 | 0/11 | 0 |
| Vehicle #122+$PGF_{2\alpha}$ (10 mg/kg) | 0/11 | 0/11 | 0 |

MA=mefenamic acid
$PGE_2$=16,16-dimethyl-$PGE_2$
$PGF_{2\alpha}$=16,16-dimethyl-$PGF_{2\alpha}$
Vehicle #122=0.25% methylcellulose solution
Prostaglandins administered in 5% ethanol, mefenamic acid administered in vehicle #122. Administration was by stomach tube.

## Claims

1. Use of a renal cytoprotective prostaglandin for the manufacture of a medicament for preventing, in a mammal, renal papillary necrosis induced by the administration of a non-steroidal anti-inflammatory compound.

2. Use according to claim 1, in which the mammal to which the non-steroidal anti-inflammatory compound is administered is not experiencing gastro-intestinal side-effects.

3. Use according to claim 1 or claim 2, in which the prostaglandin is 16,16-dimethyl-$PGE_2$.

4. Use according to claim 1 or claim 2, in which the prostaglandin is 16,16-dimethyl-$PGF_{2\alpha}$.

## Patentansprüche

1. Verwendung eines Nierenzellschutzprostaglandins zur Herstellung eines Medikamentes zur Verhütung von Renalpapillarnekrose in Säugetieren, hervorgerufen durch die Verabreichung einer nichtsteroiden, entzündungsverhütenden Verbindung.

2. Verwendung nach Anspruch 1, wobei das Säugetier, dem die nichtsteroide, entzündungsverhütende Verbindung verabreicht wird, keine gastrointestinalen Nebenwirkungen aufweist.

4

3. Verwendung nach Anspruch 1 oder Anspruch 2, wobei das Prostaglandin 16,16-Dimethyl-$PGE_2$ ist.
4. Verwendung nach Anspruch 1 oder Anspruch 2, wobei das Prostaglandin 16,16-Dimethyl-$PGF_2$ ist.

**Revendications**

1. Utilisation d'une prostaglandine à effet cytoprotecteur rénal pour la production d'un médicament destiné à éviter, chez un mammifère, la nécrose papillaire rénale induite par l'administration d'un composé anti-inflammatoire non stéroidien.

2. Utilisation suivant la revendication 1, dans laquelle le mammifère auquel le composé antiinflammatoire non stéroidien est administré n'éprouve pas d'effets secondaires gastro-intestinaux.

3. Utilisation suivant la revendication 1 ou 2, dans laquelle la prostaglandine est la 16,16-diméthyl-$PGE_2$.

4. Utilisation suivant la revendication 1 ou 2, dans laquelle la prostaglandine est la 16,16-diméthyl-$PGF_{2\alpha}$.